# EUROPEAN PATENT APPLICATION

(11) **EP 2 705 858 A1**
(43) Date of publication of application: **12.03.2014**
(21) Application number: 12460071.9
(22) Date of filing: 04.10.2012
(51) Int. Cl.: A61L 2/10, A23L 3/28, A61L 2/24, B65B 55/08, B27K 5/00

(54) **Disinfection tunnel, in particular for surface disinfection of objects**

(30) Priority: 08.09.2012 PL 40031212
(71) Applicant: Pol-Pack Service S.A., 31-358 Krakow (PL)
(72) Inventor: Seidler, Piotr, 32-082 Bolechowice (PL)
(74) Representative: Karczmitowicz, Teresa Ewa

(57) **Abstract**

The subject of claimed invention is a disinfection tunnel, in particular for surface disinfection of undurable objects, especially the objects made of materials which cannot be treated with chemicals, like wooden pallets, cardboard or paperboard separators, containers, boxes, bags, bottles and others. The disinfection tunnel has a roller conveyor (1) driven by a driving unit with a speed regulated by a controller, optionally with an inverter, and which is spreading out along the housing (2). The housing is divided into three chambers, an inlet chamber (A), an irradiation chamber (B) and an outlet chamber (C). These chambers are separated by the protective internal flexible curtains (7b, 7c). The inlet chamber (A) and the outlet chamber (C) constitute the buffer zones protecting from UV radiation and are provided with the protective external flexible curtains (7a, 7d). The curtains can be made from overlapping rubber strips. The housing (2) can be assembled from the modules (D). The UV light sources (4) are installed in the irradiation chamber (B). The device is provided with an automatic UV light detection system switching-off the UV light sources (4) if any one of the protective internal flexible curtains (7b, 7c) is not closed. Invented disinfection tunnel can be connected to the production lines, in particular in food processing plants or in the packages producing plants.

## Description

The invention relates to a disinfection tunnel for cleaning of microbial contamination a surface of reusable packaging materials and objects with UV radiation, especially the objects made of materials which cannot be treated with chemicals, like wooden pallets, cardboard or paperboard separators, containers, boxes, bags, bottles, and others. In particular, the invention relates to the food industry, where mostly only one disinfecting agent is used, i.e. the UV radiation.

Ultraviolet part of the electromagnetic spectrum was discovered in the early nineteenth century (J. Ritter, W.H. Wollaston.) and a century later it has been used for industrial purposes. Since in 1916, in the U.S., drinking water was for the first time disinfected with short-wavelength UV irradiation, research on disinfecting qualities of ultraviolet radiation has been progressing. Ultraviolet is a range of electromagnetic waves with a length of from 10 to 380 nanometers. This range is further divided into four sub-ranges, A (long waves), B (medium length waves), C (short waves), and according to some sources: D (very short waves):
UVA, wavelength of 320-400 nm,
UVB, wavelength of 280-320 nm,
UVC, wavelength of 200-280 nm.

It has been proven that UVC shows germicidal properties by destruction of genetic structure of vegetative forms of bacteria through disturbance in the process of DNA replication. So the UV rays are used to sterilize medical devices because of their high frequency and energy carried by the waves.

Application of properly selected time and intensity of UV radiation can lead to complete sterilization of treated environment. With lower intensity of radiation the percentage of killed bacteria decreases. Germicidal effects of UV radiation are different depending on wavelength. The strongest antibacterial properties show the wavelengths from about 230 to 275 nm (UVC range), these waves are absorbed by nucleic acids and proteins. Apart from germicidal properties, UV radiation has also an ability to inactivate specific antisera, bacterial toxins, etc.

The above mentioned properties of UV radiation are thus used for cleaning and/or decontamination of various materials which contain or may contain biological contaminants. For example a water disinfection device, known from the American patent application US20060471505 (A1), consists of the irradiation chamber and a source of UV radiation mounted in the middle. Disinfected surface is washed by a gas or liquid, e.g. water vapor, deionized water with or without additives, or aqueous solutions of cleaning agents. Disinfection process runs continuously, and the fluid flows into the device through the inlet nozzle and after irradiation flows out through the outlet nozzle. A UVC sensor is usually placed in the middle of the irradiation chamber. This device is appropriate for disinfection of single object at a time.

In Japanese description of the invention JP11347506 (A) a cleaning device using UV radiation and ozone is disclosed. It has a cleaning chamber enclosed in the armor chamber, with two zones on both sides of the cleaning chamber , provided with an exhaust part for discharging the ozone leaking from the cleaning chamber. High concentrations of ozone can be produced in the cleaning chamber by UV radiation generated by the UV lamps, and excess ozone is removed from the device. Cleaned items are transported within the device on the rollers. In this device only thin, flat and relatively small objects can be treated with disinfecting agents. The UV light falls only on upper surfaces of cleaned objects. Both ozone and scattered UV light can leak to the outside through open inlet and outlet slits.

Another Japanese patent application JP2011235210 (A) also discloses an ozone-UV cleaning device, consisting of two, facing each other UV irradiation units contained in the irradiation chamber. A grip for insertion of decontaminated objects into the chamber is placed between the UV irradiation units. Additionally, the air as a source of ozone, an additional disinfectant, is supplied to the device. An exhaust pump for ozone and fumes generated in the chamber is located in lower part of the device. Described device is suitable for disinfection of singular, thin and light objects, which can be hanged on the grip and inserted through an open slit. Likewise hereinbefore, this invention does not solve the problem of acceptable protection from ozone and UV light, harmful to the environment.

A washing system of plastic or metal, returnable and reusable packages is known from the American description of the invention US20080664071 (A1). The system consists of a sealed tubular tunnel, made of synthetic material. The tube is positioned horizontally and contains a cleaning tunnel, a water container and a kind of a belt conveyor provided with carriers and adjustable guides for packages, where the cleaning tunnel may be elongated by adding further tubular elements. The operations of washing, rinsing and drying are carried out in the tunnel.

All of the above solutions except of the latter are joining diverse disinfecting and purifying agents, i.e. UV radiation, ozone and water. These methods are expensive because of use of agents additional to UV light. The ozone is dangerous because of its carcinogenic properties and should be neutralized after usage. Polluted water should be cleaned and the waste neutralized chemically and biologically. This generates undesirable costs.

In an international patent application WO2004064874 A2 a disinfection system based only on UV radiation is disclosed. The disinfected objects are transported on a belt conveyor. The conveyor belt is interleaved between the upper and lower rollers. It passes first over an upper roller, then under an adjacent lower roller, and then over another upper roller to form a trough. The UV radiation unit is supported in the trough, disposed so that the disinfected objects passing along the conveyor belt will pass over the UV radiation unit without coming into contact with it. As the conveyor belt moves the objects through the treatment section, over and past the troughs and the UV radiation units disposed in the troughs, these UV radiation units treat the undersides of these objects. The UV radiation units in the troughs also treat the belt while it is free from treated objects in each trough. Upper or side UV radiation units may also be provided for 360 degree disinfection.

The problem of disinfection of large objects like the wooden pallets is not solved by application of the device disclosed in the document WO2004064874 A2 for three main reasons. Firstly, the belt should be easily interleaved between the rollers, however the belts used for transporting the objects like the pallets, often with protruding bare nails, splinters and sharp edges of the wood, should be mechanically resistant and relatively stiff. Such belts are not so much elastic and cannot be interlaced between relatively thin rollers. Besides, continuous deformations of the belt on thin rollers significantly reduce its durability because of strain ageing. Secondly, the belt has to be exposed to UV radiation, because otherwise disinfected objects will be again contaminated by the belt. It is commonly known, that UV radiation, in particular in mostly energetic UVC range which is the best for disinfection, destroy also other organic polymers like various rubbers used for production of conveyor belts and accelerates the rubber light ageing. Therefore efficient disinfection is very expensive because of necessity of frequent exchange of conveyor belts or application of the belts made from special, UV-resistant rubbers. Thirdly, in this solution no any means for protection from the UV radiation are proposed or even suggested. This is very important technical problem, because in case of disinfection of large objects like the pallets with intense UV radiation the risk of irradiation of the staff is very high.

All of the above technical problems are solved by claimed invention. A disinfection tunnel is proposed, which is provided with a roller conveyor. Disinfection process is based on UV radiation only, which solves the problem of ozone-caused pollution. Disinfected large objects are transported on transporting rollers instead of the conveyor belt. The tunnel is made of a material reflecting and scattering the UV radiation, e.g. metal sheet or foil, thus maximizing effectiveness of the disinfection procedure. Invented disinfection tunnel is equipped with multilevel protective means, which secure the environment and in particular the people around the device from harmful UV radiation. Invented device is safe, simple in operation and maintenance, with minimized number of mechanically or radio-sensitive parts, and low susceptibility to chemical attack or overheating. An important feature of the device is a possibility to connect it with a technological line producing packaging containers for the food industry.

A disinfection tunnel, in particular for surface disinfection of undurable objects, with a conveyor and the sources of UV radiation mounted inside a housing, according to the invention is **characterized in that** the conveyor is a roller conveyor driven by a driving unit with controlled speed and which is spreading out along the housing. Double protection, preventing of penetration of UV radiation outside the tunnel is achieved by creating the buffer zones, secured with protective curtains. The housing is divided into three chambers, an inlet chamber, an irradiation chamber and an outlet chamber, which are separated by the protective internal flexible curtains. The inlet chamber and the outlet chamber are provided with the protective external flexible curtains. The sources of UV radiation are the UV light sources installed in the irradiation chamber. The disinfection tunnel is provided with an automatic UV light detection system switching-off the UV light sources if any one of the protective internal flexible curtains is not closed.

In preferred embodiment of the invention the UV light sources are mounted on the side walls and/or the ceiling of the irradiation chamber. The UV light sources can also be mounted between the rollers of the roller conveyor inside the irradiation chamber.

The UV light sources radiate at wavelengths of from 250 nm to 258 nm, preferably from 253 nm to 254 nm Radiation of this wavelength results in permanent inactivation of bacteria, viruses, mold and spores. The process involves absorption of radiation by the gene structure of DNA, leading to its distortion, thereby preventing the replication process. In recommended embodiment these are the fluorescent lamps.

The disinfection tunnel according to the invention has been designed for the decontamination/sterilization of a particular type of objects made of undurable materials such as wooden pallets and the objects made of a cardboard. The study showed that for these types of materials, the UV light sources should have a power of 10 W to 100 W. Kind, power and the number of applied UV light sources depend on the type of surface of the material to be irradiated.

Preferably a distance between the UV light sources mounted on the side walls and/or the ceiling of the irradiation chamber is between 1 m and 3 m, which ensures a uniform distribution of radiation in the whole irradiation chamber. For safety reasons the UV light sources are enclosed in protective shields with UV-transparent windows, preferably made of glass, PMMA, PC or PVC. Instead or additionally, the UV light sources installed between the rollers can be protected with UV-transparent glass, PMMA, PC or PVC plates.

The housing is made of metal sheet or metal foil, and/or plastic, preferably PVC or PE.

The driving unit comprises an electric drive and a transmission. The driving power is transmitted from the electric drive through the transmission to the rollers by at least one driving chain. The transportation speed of the UV-treated objects on the roller conveyor is controlled with a controller connected to the driving unit. Advantageously, the controller is connected to the driving unit through an inverter. In one embodiment the controller is provided with the means for manual control. These means for manual control comprise a display screen, a keyboard, a mouse or joystick or a touchpad, and/or other data input/output devices, a data storage unit and a processor.

Preferably the housing is assembled from the modules. Adjacent modules are joined together, preferably with clips. For UV treatment of relatively big objects like the pallets the modules have length of between 1.5 m and 3 m, and/or have width of between 1.5 m and 3 m. For longer mechanical durability the modules are made of metal sheet, preferably steel sheet, of thickness between 2 mm and 4 mm.

Conveyed object like a pallet enters the irradiation chamber through the buffer zone, i.e. through the inlet chamber, allowing for completely covering the protective external flexible curtain, before moving the item towards the protective internal flexible curtain, opening the irradiation chamber. The length of the buffer zone depends on the length of the object to be irradiated. In preferred embodiment the inlet chamber and the outlet chamber have lengths of between 1 m and 2 m.

The protective external flexible curtains and/or the protective internal flexible curtains consist of the rubber strips assembled with overlapping longer sides, tightly covering the entrance and exit to the chambers. The rubber strips have width of between 7 cm and 10 cm, and thickness of between 2 mm and 10 mm.

Highest safety of the disinfection tunnel according to the invention is secured by the automatic UV light detection system, which in one embodiment is provided with the sensors attached to the protective internal flexible curtains for securing from opening of the protective internal flexible curtain during UV light emission by the UV light sources. In another embodiment, which can be realized jointly with the former one, the automatic UV light detection system is provided with the sensors attached to the protective internal flexible curtains for switching-off the UV light sources in case of opening of the protective internal flexible curtain. It is not possible to run the UV light sources in the case of open curtains.

The disinfection tunnel can be provided optionally with the guides of width preferably adjustable to the size of treated object, which allows for easy conveying of disinfected objects of different widths.

The housing of the disinfection tunnel advantageously has an internal lining of high reflection index for UV light, preferably made from aluminum sheet or aluminum foil. For uniformity of treated object irradiation with direct and scattered UV light, the surface of internal lining is mat or rough.

A spacing of adjacent rollers of between 5 cm and 20 cm is appropriate for relatively big objects.

In the disinfection tunnel according to the invention a space between the upper side of the roller conveyor, i.e. the upper plane of the rollers, and the ceiling of the housing is between 0.5 m and 1.5 m. The disinfection tunnel height from the ground ranges from 1.5m to 3 m.

For easy and safe maneuvering with large objects, in some embodiments the roller conveyor is longer than the housing. The roller conveyor may protrude from both inlet and outlet side of the housing. For special purposes the roller conveyor projects from one side of the housing. The other side of the housing can be provided with the means connecting it to a production line.

The disinfection tunnel as described hereinbefore can be applied in a production line, in particular in the food industry.

The object treated with UV radiation is fed on a roller conveyor and automatically advanced to the inlet chamber, and then to the irradiation chamber, and finally to the outlet chamber. After leaving the outlet chamber the object is sterile and ready for the next use.

During decontamination of the objects with use of the disinfection tunnel according to the invention the by-products of disinfection are not formed, since the radiation intensity required for disinfection is less than necessary for the photochemical reactions.

The treatment with UV radiation of the transport packages such as wooden pallets and cardboard spacers used in the food industry and food processing plants for packaging food products, and in the plants producing packages for the food industry for packaging of their products, contributes to increase hygienic safety and eliminates a possibility of contamination of the products and people.

An advantage of UV treatment with use of the disinfection tunnel according to the invention is a decontamination of any micro-organisms. It allows for multiple use of transport packages in considerably longer time. They can be much longer to re-used for packaging, with assured purity of these packages. This may reduce the introduction of new transport packages as well as the amount of packages directed to recycling. This clearly contributes to the environment protection.

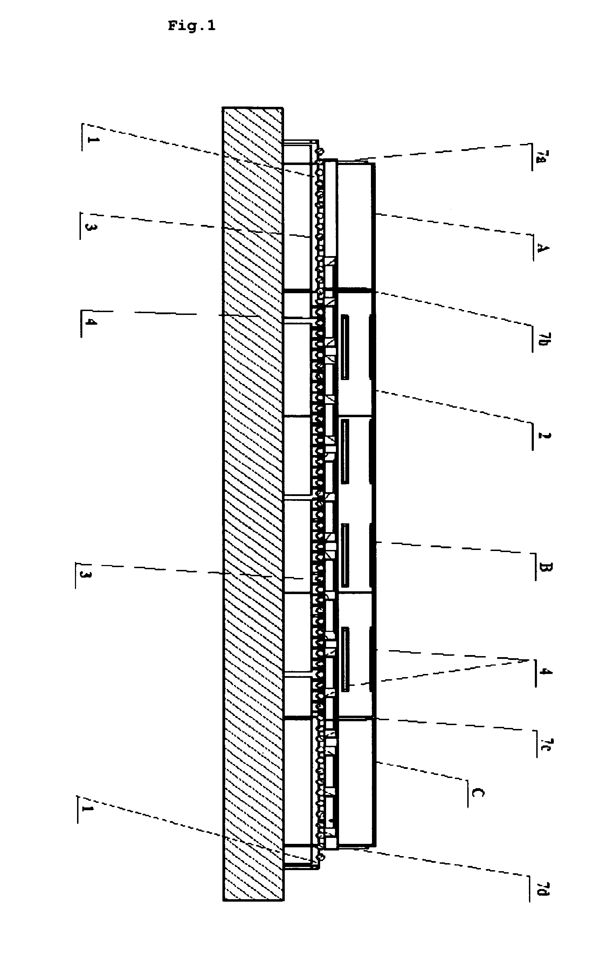

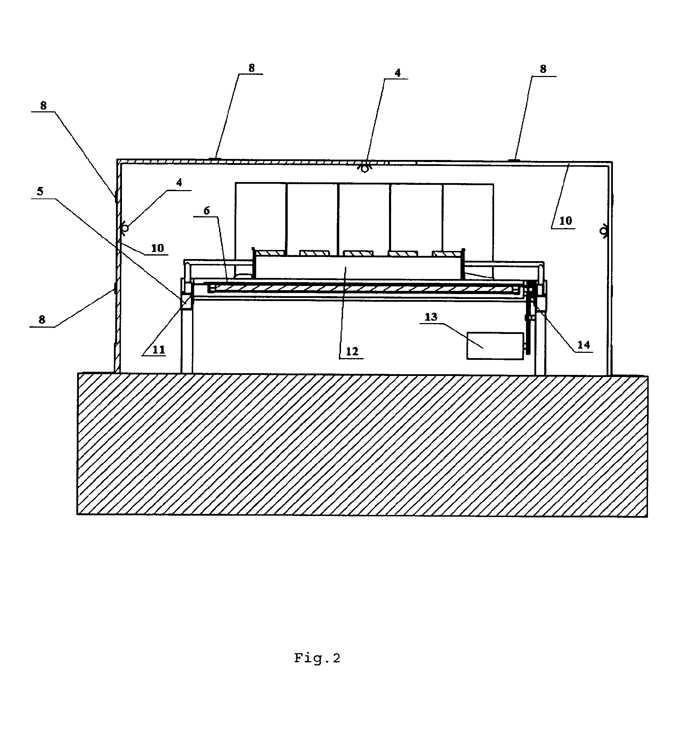

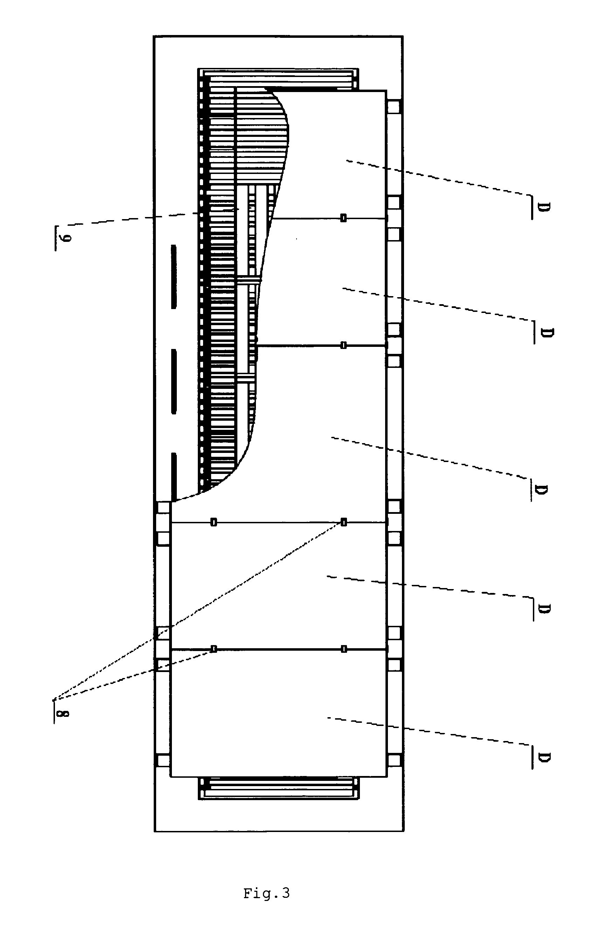

The invention is shown in the embodiment on the drawing, where Fig. 1 shows the disinfecting tunnel in longitudinal section, Fig. 2 shows a cross section of the disinfecting tunnel, and Fig. 3 shows a top view of the disinfecting tunnel.

The disinfection tunnel according to the invention consists of the roller conveyor mounted in the housing 2 made of steel sheet. It is divided into the inlet chamber A, the irradiation chamber B and the outlet chamber C. All of the three chambers A, B and C are made of the modules D connected together with the clips 8. On the entrance and exit sides of the housing 2, i.e. on the entrance side of the inlet chamber A and on the exit side of the outlet chamber C the protective external flexible curtains 7a and 7d are installed. Also on both sides of the irradiation chamber B the protective internal flexible curtains 7b and 7c are mounted. These curtains are made from rubber in the form of overlapping strips 10 cm wide and 10 mm thick. The roller conveyor 1 is longer than the housing 2, which facilitates introduction of the object into the disinfection tunnel. Along entire length of the irradiation chamber B, the UV light sources 4 are mounted between the rollers 3 to the frame 5 of the roller conveyor 1 with the fasteners 11.

The UV radiating fluorescent tubes 4 are placed below the upper plane of the rollers 3 in the roller conveyor 1, in the shields 6 protecting them against damage and contamination. A lack of any contact between the UV light sources and treated objects 12 is thus secured.

Internal sides of the walls and ceiling of the irradiation chamber B is lined with a material 10 highly reflective in the UV range of the spectrum, like the aluminum foil.

The rollers 3 of the roller conveyor 1 are driven by the electric motor with a gear 13 through the transmission 14. Driving power is transmitted by a chain. The rotational speed of the electric motor with a gear 13 is controlled with an inverter which makes possible to increase and reduce the transportation speed on the roller conveyor 1.

Above the rollers 3, on both sides of the roller conveyor 1 are placed adjustable side guides 9 that allow for proper handling of the material along the housing 2.

The length of the roller conveyor 1 and of the housing 2 is adjusted to the desired performance of the device.

## Claims

1. A disinfection tunnel, in particular for surface disinfection of undurable objects, with a conveyor and the sources of UV radiation mounted inside a housing, **characterized in that** the conveyor is a roller conveyor (1) driven by a driving unit with controlled speed and spreading out along the housing (2) which is divided into three chambers, an inlet chamber (A), an irradiation chamber (B) and an outlet chamber (C), which are separated by the protective internal flexible curtains (7b, 7c), while the inlet chamber (A) and the outlet chamber (C) are provided with the protective external flexible curtains (7a, 7d), and the sources of UV radiation are the UV light sources (4) installed in the irradiation chamber (B), and also **characterized in that** it is provided with an automatic UV light detection system switching-off the UV light sources (4) if any one of the protective internal flexible curtains (7b, 7c) is not closed.

2. The disinfection tunnel according to claim 1, **characterized in that** the UV light sources (4) are mounted on the side walls and/or the ceiling of the irradiation chamber (B).

3. The disinfection tunnel according to claim 1 or 2, **characterized in that** the UV light sources (4) are mounted between the rollers (3) of the roller conveyor (1) inside the irradiation chamber (B).

4. The disinfection tunnel according claim 1 or 2, or 3, **characterized in that** the UV light sources (4) radiate at wavelengths of from 250 nm to 258 nm, preferably from 253 nm to 254 nm.

5. The disinfection tunnel according to any of preceding claims, **characterized in that** the UV light sources (4) are the fluorescent lamps of power from 10 W to 100 W.

6. The disinfection tunnel according to any of preceding claims, **characterized in that** a distance between the UV light sources (4) mounted on the side walls and/or the ceiling of the irradiation chamber (B) is between 1 m and 3 m.

7. The disinfection tunnel according to any of preceding claims, **characterized in that** the UV light sources (4) are enclosed in protective shields with UV-transparent windows, preferably made of glass, PMMA, PC or PVC.

8. The disinfection tunnel according to any of preceding claims, **characterized in that** the housing (2) is made of metal sheet or metal foil, and/or plastic, preferably PVC or PE.

9. The disinfection tunnel according to any of preceding claims, **characterized in that** the driving unit comprises an electric drive (13) and a transmission (14).

10. The disinfection tunnel according claim 9, **characterized in that** driving power is transmitted from the electric drive (13) through the transmission (14) to the rollers (3) by at least one driving chain.

11. The disinfection tunnel according to any of preceding claims, **characterized in that** transportation speed of the UV-treated objects on the roller conveyor (1) is controlled with a controller connected to the driving unit.

12. The disinfection tunnel according to claim 11, **characterized in that** the controller is connected to the driving unit through an inverter.

13. The disinfection tunnel according to claim 11 or 12, **characterized in that** the controller is provided with the means for manual control.

14. The disinfection tunnel according to claim 13, **characterized in that** the means for manual control comprise a display screen, a keyboard, a mouse or joystick or a touchpad, and/or other data input/output devices, a data storage unit and a processor.

15. The disinfection tunnel according to any of preceding claims, **characterized in that** the housing (2) is assembled from the modules (D), where adjacent modules (D) are joined together, preferably with clips (8).

16. The disinfection tunnel according to claim 15, **characterized in that** the modules (D), have length of between 1.5 m and 3 m, and/or have width of between 1.5 m and 3 m.

17. The disinfection tunnel according to claim 15 or 16, **characterized in that** the modules (D) are made of metal sheet, preferably steel sheet, of thickness between 2 mm and 4 mm.

18. The disinfection tunnel according to any of preceding claims, **characterized in that** the inlet chamber (A) and the outlet chamber (C) have lengths of between 1 m and 2 m.

19. The disinfection tunnel according to any of preceding claims, **characterized in that** the protective external flexible curtains (7a, 7d) and/or the protective internal flexible curtains (7b, 7c) consist of the rubber strips assembled with overlapping longer sides.

20. The disinfection tunnel according to claim 19, **characterized in that** the rubber strips have width of between 7 cm and 10 cm, and thickness of between 2 mm and 10 mm.

21. The disinfection tunnel according to any of preceding claims, **characterized in that** the automatic UV light detection system is provided with the sensors attached to the protective internal flexible curtains (7b, 7c) for switching-off the UV light sources (4) in case of opening of the protective internal flexible curtain (7b, 7c).

22. The disinfection tunnel according to any of preceding claims, **characterized in that** it is provided with the guides (9), of width preferably adjustable to the size of treated object.

23. The disinfection tunnel according to any of preceding claims, **characterized in that** the housing (2) has an internal lining (10) of high reflection index for UV light, preferably made from aluminum sheet or aluminum foil.

24. The disinfection tunnel according to claim 24, **characterized in that** the surface of internal lining (10) is mat or rough.

25. The disinfection tunnel according to any of preceding claims, **characterized in that** spacing of adjacent rollers (3) is between 5 cm and 20 cm.

26. The disinfection tunnel according to any of preceding claims, **characterized in that** space between the upper side of the roller conveyor (1) and the ceiling of the housing (2) is between 0.5 m and 1.5 m.

27. The disinfection tunnel according to any of preceding claims, **characterized in that** the roller conveyor (1) is longer than the housing (2).

28. The disinfection tunnel according to any of preceding claims, **characterized in that** the roller conveyor (1) projects from one side of the housing (2) while the other side of the housing (2) is provided with the means connecting it to a production line.

29. Application of the disinfection tunnel as described in any of preceding claims in a production line, in particular in the food industry.
